# FASCICULE DE BREVET EUROPEEN

(11) **EP 1 481 653 B1**
(45) Date de publication et mention de la délivrance du brevet: **30.11.2005**
(21) Numéro de dépôt: 04356082.0
(22) Date de dépôt: 27.05.2004
(51) Int. Cl.: A61F 2/38

(54) **Prothèse de coude**
Ellenbogengelenkprothese
Elbow prosthesis

(30) Priorité: 28.05.2003 FR 0306519
(43) Date de publication de la demande: 01.12.2004
(73) Titulaire: Tornier, 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 0 913 133
- EP-A- 1 051 954
- US-A- 4 383 337

## Description

L'invention a trait à une prothèse de coude comportant au moins un composant huméral et un composant cubital.

Elle concerne plus particulièrement une prothèse de coude dont le composant cubital est adapté pour recevoir un élément de verrouillage qui forme une surface articulaire prolongeant celle du composant cubital, les surfaces articulaires de l'élément de verrouillage et du composant cubital étant aptes à être disposées ensemble autour d'une partie au moins d'une autre surface d'articulation appartenant à un composant huméral. Une prothèse de ce type est connue de EP-A-1 051 954.

Dans une telle prothèse, l'élément de verrouillage est maintenu en position sur le composant cubital grâce à une vis introduite dans un logement traversant de cet élément et serrée dans un taraudage prévu sur le composant cubital. Il s'avère en pratique que le positionnement de l'élément de verrouillage sur le composant cubital peut ne pas être optimal lors du serrage d'une telle vis, ce qui peut amener à un positionnement en biais de la vis par rapport au taraudage, au point que le filet de la vis ou le taraudage peut être faussé, empêchant ainsi le montage de l'élément de verrouillage. En outre, la vis précitée sert à la fois à amener l'élément de verrouillage en position correcte par rapport au composant cubital et à assurer le blocage de ces deux éléments l'un par rapport à l'autre. Il en résulte qu'un tel composant cubital doit le plus souvent être posé alors que l'articulation du coude est en flexion maximale, ce qui complexifie le travail du chirurgien. Il arrive même qu'une telle pose en flexion maximale ne soit pas possible, notamment en cas d'obésité prononcée du patient.

C'est à ces inconvénients qu'entend plus particulièrement remédier l'invention en proposant une prothèse de coude dont la pose est facilitée, lorsqu'elle comprend un élément de verrouillage tel que mentionné ci-dessus.

Dans cet esprit, l'invention concerne une prothèse de coude comprenant un composant huméral, formant une première surface d'articulation et un composant cubital formant une seconde face d'articulation apte à être disposée autour d'une partie de la première surface d'articulation et à pivoter autour d'un axe longitudinal de cette surface, le composant cubital étant pourvu de moyens de montage d'un élément de verrouillage formant une troisième surface d'articulation prolongeant la seconde surface d'articulation et apte à être également disposée autour d'une partie de la première surface d'articulation. Cette prothèse est caractérisée en ce que les moyens de montage précités comprennent des moyens de guidage adaptés pour coopérer avec des moyens complémentaires prévus sur l'élément de verrouillage pour guider cet élément en translation par rapport au composant cubital, jusqu'à une position où des moyens de blocage prévus respectivement sur l'élément de verrouillage et sur le composant cubital peuvent être mis en oeuvre pour immobiliser l'élément de verrouillage sur le composant cubital, ces moyens de blocage ne pouvant être mis en oeuvre que dans cette position.

Grâce à l'invention, les moyens de guidage assurent un positionnement adéquat de l'élément de verrouillage par rapport au composant cubital avant que les moyens de blocage ne soient mis en oeuvre pour réaliser une immobilisation efficace de l'élément de verrouillage par rapport au composant cubital. En d'autres termes, les moyens de blocage ne servent pas à amener l'élément de verrouillage dans une configuration permettant son blocage, cette fonction étant dévolue aux moyens de guidage qui peuvent être positionnés et configurés pour assurer au mieux cette fonction.

En outre, une prothèse de coude peut incorporer une ou plusieurs des caractéristiques suivantes, prises dans toute combinaison techniquement admissible :
- Les moyens de blocage comprennent une vis et un taraudage complémentaires prévus respectivement sur l'élément de verrouillage et sur le composant cubital, ou réciproquement, la direction de translation relative entre l'élément de verrouillage et le composant cubital telle qu'imposée par les moyens de guidage étant non parallèle à l'axe longitudinal du taraudage et/ou de la vis précitée.
- Les moyens de guidage comprennent au moins un relief faisant saillie par rapport à une partie principale du composant cubital, alors que les moyens complémentaires prévus sur l'élément de verrouillage sont aptes à interagir avec ce relief par coopération de formes. Dans ce cas, le relief précité peut être un nez, alors que les moyens complémentaires comprennent une fourche dont deux branches définissent entre elles un volume de réception et de coulissement de ce nez.
- Les moyens de guidage et les moyens de blocage sont ménagés au voisinage de deux bords opposés de la seconde surface d'articulation du composant cubital, alors que les moyens complémentaires et les moyens de blocage prévus sur l'élément de verrouillage sont ménagés sur deux bords opposés de cet élément.
- Les moyens de blocage comprennent une vis et un taraudage complémentaires prévus respectivement sur l'élément de verrouillage et sur le composant cubital, ou réciproquement, alors qu'il est prévu des moyens de blocage en rotation de cette vis. Ainsi, la tête de la vis peut être à section non circulaire, alors que la pièce contre laquelle porte cette tête forme un espace de réception de cette tête, avec contact entre la surface de cette pièce et la surface externe de la tête. Plus particulièrement, la tête de la vis peut être pourvue de crans aptes à pénétrer superficiellement dans la surface de la pièce précitée. En variante, la pièce contre laquelle porte la tête de la vis peut être pourvue d'une patte repliable en configuration de blocage de cette tête. Dans ce cas, la tête de la vis peut être à profil polygonal, avec des faces dont la largeur est supérieure à la largeur de la patte repliable.

L'invention sera mieux comprise et d'autres avantages de celle-ci apparaîtront plus clairement à la lumière de la description qui va suivre d'un mode de réalisation d'une prothèse conforme à son principe, donnée uniquement à titre d'exemple et faite en référence aux dessins annexés dans lesquels :
- la figure 1 est une vue en perspective éclatée d'une prothèse de coude conforme à l'invention ;
- la figure 2 est une coupe longitudinale du composant cubital et de l'élément de verrouillage en cours de montage ;
- la figure 3 est une coupe analogue à la figure 2 alors que l'élément de verrouillage est monté sur le composant cubital ;

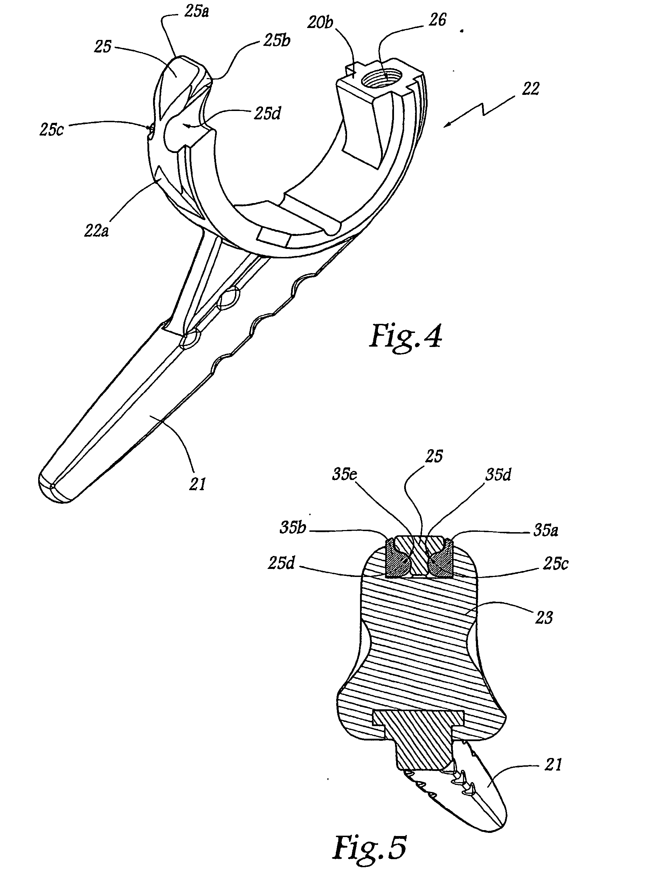

- la figure 4 est une vue en perspective de la seule partie métallique du composant cubital et
- la figure 5 est une coupe selon la ligne V-V à la figure 3 ;

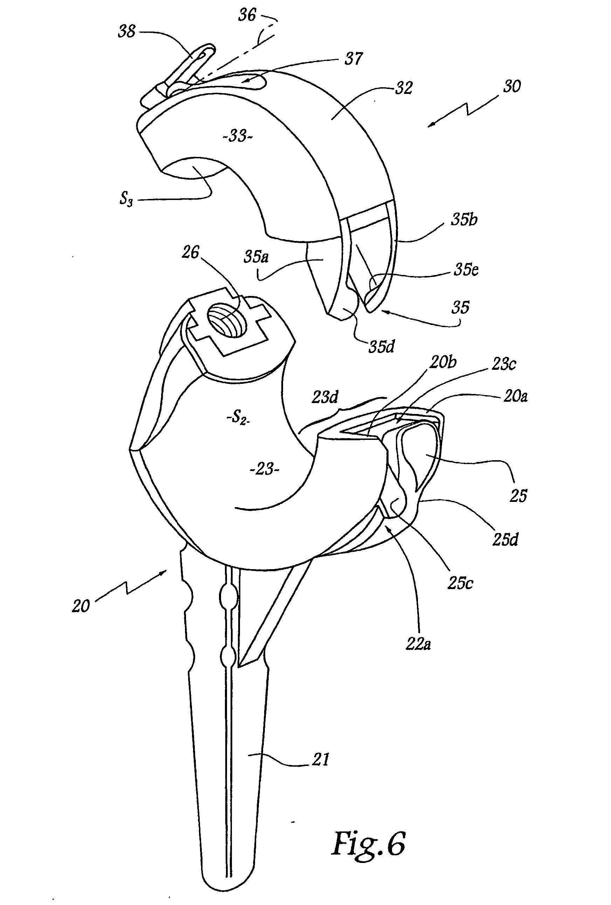

- la figure 6 est une vue en perspective à plus grande échelle du composant cubital et de l'élément de verrouillage de la prothèse.

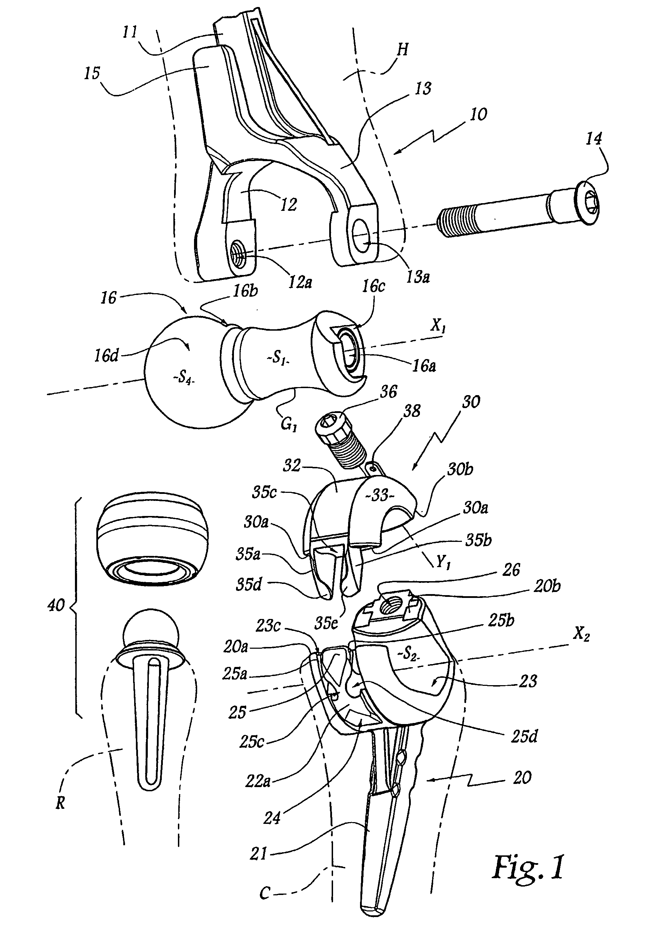

La prothèse visible aux figures 1 à 3 comprend un composant huméral 10 comportant une tige 11 destinée à être enfoncée dans le canal médullaire d'un humérus H et se prolongeant par deux pattes 12 et 13 percées chacune d'un orifice 12a, 13a de passage d'une vis canulée 14 formant axe. Le caractère creux de la vis 14 permet le passage de fils de suture. L'orifice 12a est taraudé, ce qui permet le vissage de la vis 14. La tige 11 se prolonge également par une troisième patte 15 destinée à venir en appui contre la corticale de l'humérus.

Une pièce allongée 16 est pourvue d'un perçage central 16a dont les dimensions permettent de recevoir la vis 14, ce qui permet de monter la pièce 16 entre les branches 12 et 13. La pièce 16 est pourvue de deux surfaces d'extrémité 16b et 16c prévues pour venir respectivement au contact des surfaces en regard des pattes 12 et 13.

Entre les surfaces 16b et 16c, la pièce 16 est globalement cylindrique, à base circulaire, et forme une surface S₁ d'articulation également globalement cylindrique dont la génératrice G₁ est courbe et concave, en ce sens que le diamètre de la surface S₁ est minimal dans la partie centrale de la pièce 16 comprise entre les surfaces 16b et 16c. On note X₁ l'axe de symétrie de la surface S₁.

La pièce 16 se prolonge par une extension 16d dont la surface externe S₄ est convexe et qui coiffe la patte 12 lorsque la pièce 16 est montée entre les pattes 12 et 13.

L'ensemble du composant huméral 10 est métallique.

Le composant cubital 20 comprend une tige métallique 21 destinée à être insérée dans le canal médullaire du cubitus C et qui est monobloc avec une patte 22 également métallique de forme concave et dont la surface intérieure est revêtue d'une garniture 23 réalisée dans un matériau adapté au frottement avec la pièce métallique 16, par exemple en polyéthylène. La garniture 23 est montée sur la patte 22 par coopération de formes. Une goupille 27 permet d'immobiliser les éléments 22 et 23 l'un par rapport à l'autre.

La surface interne de la garniture 23 forme une surface d'articulation S₂ de forme complémentaire de la surface S₁. On note X₂ l'axe central des éléments 22 et 23.

Les diamètres respectifs des surfaces S₁ et S₂, diamètres qui sont variables le long des axes X₁ et X₂, sont choisis de telle sorte que la surface S₂ peut être disposée autour de la surface S₁, avec un léger jeu, ce qui permet une rotation des composants 10 et 20 l'un par rapport à l'autre autour des axes X₁ et X₂ qui sont alors confondus.

De plus, les profils des génératrices des surfaces S₁ et S₂ sont choisis de manière à permettre un degré de mobilité de la surface S₁ dans la surface S₂, dans le sens du varus-valgus, c'est-à-dire autour d'un axe perpendiculaire à X₁ et X₂.

Un élément de verrouillage 30 est destiné à être monté sur le composant 20 et comprend une armature métallique 32 ainsi qu'une garniture 33 immobilisées l'une par rapport à l'autre par clipsage. La garniture 33 est avantageusement réalisée dans le même matériau que la garniture 23 et forme une surface d'articulation concave S₃ destinée à prolonger la surface S₂ et à entourer avec elle la surface S₁.

Pour faciliter le montage de l'élément 30 sur le composant 20, ce composant est pourvu, dans le prolongement de la surface externe 24 de la patte 22, d'un relief 25 en forme de nez ou de bec qui fait saillie au-delà de la partie 22a de la patte 22 recouverte par la garniture 23.

Par ailleurs, l'armature 32 de l'élément 30 se prolonge au-delà de sa partie 32a recouverte par la garniture 33, par une extension en forme de fourche 35, dont les deux branches 35a et 35b définissent entre elles un volume 35c de coulissement et de réception du nez 25 lors du montage de l'élément 30 sur le composant 20.

Le nez 25 est disposé dans un espace 23c défini par la garniture 23 qui se prolonge au-delà de la partie 22a de la patte 22 qu'elle recouvre. Dans sa partie 23d qui s'étend en porte à faux par rapport à la partie 22a, la garniture a une section transversale globalement en U. L'espace 23c est défini au centre de ce U.

Le nez 25 a une largeur décroissante en direction du fond du U formé par la partie 23d. Ainsi, les côtés 25a et 25b du nez 25 divergent en s'éloignant de ce fond et se rapprochent des branches du U formé par la partie 23d. Le nez 25 a une section globalement en forme de queue d'aronde.

En outre, des rainures 25c et 25d sont ménagées de part et d'autre du nez 25. Ces nervures ont des profils leur permettant de recevoir des parties bombées 35d et 35e des branches 35a et 35b.

Les branches 35a et 35b ont ainsi une géométrie compatible avec leur introduction dans l'espace 23c, de part et d'autre du nez 25. Leurs surfaces destinées à être tournées vers le nez 25 s'écartent l'une de l'autre en allant vers l'extérieur de l'élément 30, de sorte que ces surfaces sont globalement parallèles aux côtés 25a et 25b.

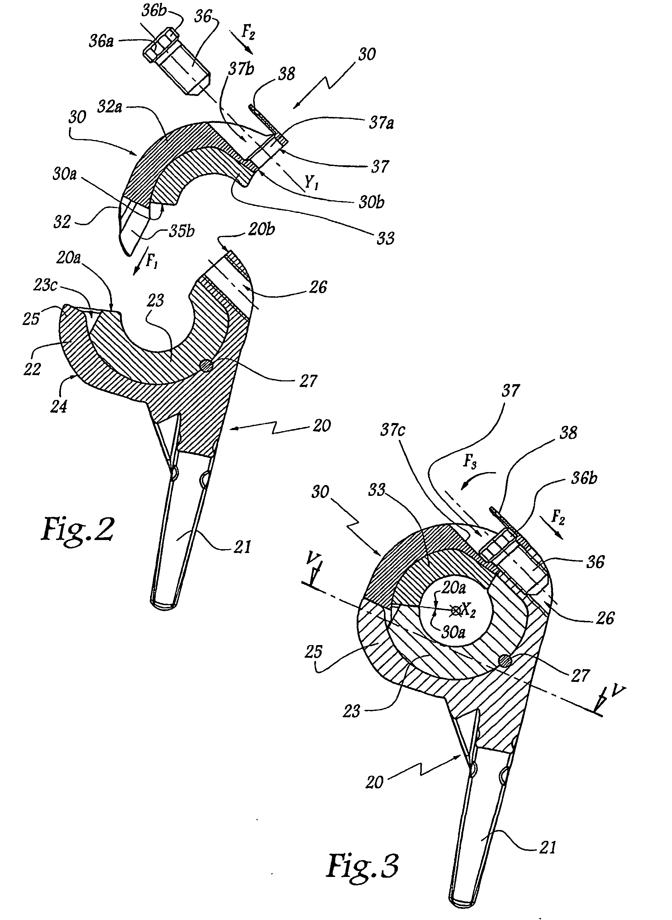

Ainsi, la direction de montage de l'élément 30 sur le composant 20 est nécessairement celle indiquée par la flèche F₁ à la figure 2, cette direction étant imposée par la coopération de formes entre le nez 25 et la fourche 35. En effet, comme indiqué ci-dessus, les sections transversales de la nervure 25 d'une part et des parties intérieures des branches 35 sont choisies pour imposer cette direction de coulissement.

Par ailleurs, le composant 20 est pourvu d'un taraudage 26 de réception d'une vis 36 qui traverse un logement traversant 37 prévu à cet effet dans l'élément 30.

Le taraudage 26 et le logement 37 sont configurés de telle sorte qu'ils sont alignés, selon une direction Y₁ perpendiculaire à l'axe X₂, au terme du coulissement de l'élément 30 par rapport au composant 20. Ce coulissement a lieu jusqu'à ce que les garnitures 23 et 33 viennent en appui l'une contre l'autre au voisinage des organes 25 et 35, comme représenté à la figure 3. Il est alors possible de visser la vis 36 dans le taraudage 26, dans le sens de la flèche F₂ aux figures 2 et 3. Dans la configuration de la figure 3, les axes centraux de la vis 36 et du taraudage 26 sont confondus et parallèles à la direction Y₁.

La direction Y₁ n'est pas parallèle au sens de déplacement de l'élément 30 par rapport au composant 20 lors de son montage, c'est-à-dire à la flèche F₁, de telle sorte que la vis 36 ne peut être introduite dans le taraudage 26 qu'au terme du coulissement de l'élément 30 sur le composant 20 dans le sens de la flèche F₁.

Ainsi, la vis 36 sert uniquement au blocage en position de l'élément 30 sur le composant 20 et ne peut être serrée qu'une fois qu'une position correcte de l'élément 30 sur le composant 20 a été atteinte. On évite ainsi les risques de vissage en biais de la vis 36 dans le taraudage 26.

En outre, la résistance mécanique de l'ensemble formé des éléments 20 et 30 dans la configuration de la figure 3 est bien supérieure à celle pouvant être obtenue avec la prothèse connue de EP-A-1 051 954 car, une fois la vis 36 serrée, l'élément 30 est immobilisé par rapport au composant 20 sur ses deux côtés.

En effet, le nez 25 est prévu au voisinage d'un premier bord 20a du composant 20 et de la surface S₂, alors que le taraudage 26 est prévu à proximité du bord opposé 20b de ce composant. De la même façon, les éléments 35 et 37 sont respectivement prévus au voisinage des deux bords opposés 30a et 30b de l'élément 30 et de la surface S₃.

En d'autres termes, la fonction de guidage de l'élément 30 par rapport au composant 20 est obtenue au voisinage des bords 20a et 30a de ces organes, alors que la fonction de blocage est obtenue au voisinage des bords 20b et 30b.

Le logement 37 est formé d'un orifice circulaire 37a ménagé dans l'armature métallique 32 pour le passage de la tige de la vis 36, ainsi que d'une zone concave 37b ménagée dans une partie d'extrémité de la garniture 33, au voisinage du bord 30b.

La tête 36a de la vis 36 est à section externe non circulaire car elle est équipée de crans ou dents 36b périphérique prévus pour s'engager superficiellement dans la surface radiale interne 37c de la zone concave 37b, ce qui concourt à l'immobilisation en rotation de la vis 36 et assure ainsi un blocage efficace de l'élément 30 sur le composant 20.

En outre, une patte 38 prolonge l'armature 32 au voisinage du logement 37, cette patte pouvant être repliée en appui contre la tête 36a de la vis 36 lorsque celle-ci est serrée, dans le sens de la flèche F₃ à la figure 3, de telle sorte qu'elle assure une fonction d'anti-recul pour cette vis, ce qui contribue également au blocage efficace de l'élément 30 sur le composant 20.

Selon une variante non représentée de l'invention, la tête 36a peut être à profil polygonal, par exemple hexagonal ou octogonal, avec des faces planes. Dans ce cas, la largeur de la patte 38 est choisie inférieure à la largeur des faces latérales de cette tête, ce qui permet un blocage en rotation de la vis 36 par appui surfacique de l'une de ces faces contre cette patte.

Lorsque l'élément 30 est monté sur le composant 20, l'assemblage ainsi réalisé peut pivoter autour de l'axe X₁ et la réunion des surfaces S₂ et S₃ entoure la surface S₁ sur 360°.

Un composant radial apte à être monté sur le radius R est avantageusement prévu dans la prothèse conforme à l'invention, ce composant 40 définissant une surface destinée à coopérer avec la surface S₄. Ceci n'est cependant pas obligatoire.

L'invention n'est pas limitée au seul mode de réalisation représenté. Elle peut, en particulier, concerner une prothèse dans laquelle l'élément en relief faisant saillie, tel que le nez 25, est prévu sur l'élément 30, alors qu'une structure de type fourche est prévue sur le composant cubital. De même, le taraudage de réception de la vis de blocage peut être prévu sur l'élément de verrouillage, le composant cubital définissant alors un logement de réception de la vis de blocage.

Tout moyen mécanique de blocage de l'élément de verrouillage sur le composant cubital peut être envisagé dans le cadre de la présente invention.

## Revendications

1. Prothèse de coude comprenant un composant huméral formant une première surface d'articulation et un composant cubital formant une seconde surface d'articulation apte à être disposée autour d'une partie de ladite première surface d'articulation et à pivoter autour d'un axe longitudinal de ladite première surface d'articulation, ledit composant cubital étant pourvu de moyens de montage d'un élément de verrouillage qui forme une troisième surface d'articulation prolongeant ladite seconde surface d'articulation et apte à être également disposée autour d'une partie de ladite première surface d'articulation, **caractérisée en ce que** lesdits moyens de montage (25, 26) comprennent des moyens (25) de guidage adaptés pour coopérer avec des moyens complémentaires (35) prévus sur ledit élément de verrouillage (30) pour guider ledit élément de verrouillage en translation (F₁) par rapport audit composant cubital (20) jusqu'à une position (figure 3) où des moyens de blocage (36, 26) prévus respectivement sur ledit élément de verrouillage et sur ledit composant cubital peuvent être mis en oeuvre (F₂) pour immobiliser ledit élément de verrouillage sur ledit composant cubital, lesdits moyens de blocage ne pouvant être mis en oeuvre que dans ladite position.

2. Prothèse selon la revendication 1, **caractérisée en ce que** lesdits moyens de blocage comprennent une vis (36) et un taraudage (26) complémentaires prévus respectivement sur ledit élément de verrouillage (30) et sur ledit composant cubital (20), ou réciproquement, la direction de translation relative (F₁) entre ledit élément et ledit composant telle qu'imposée par lesdits moyens de guidage (25, 23) étant non parallèle à l'axe longitudinal (Y₁) dudit taraudage et/ou de ladite vis.

3. Prothèse selon l'une des revendications précédentes, **caractérisée en ce que** lesdits moyens de guidage comprennent au moins un relief (25) faisant saillie par rapport à une partie principale (22a) dudit composant cubital (20), alors que les moyens complémentaires (35) prévus sur ledit élément de verrouillage (30) sont aptes à interagir avec ledit relief par coopération de formes.

4. Prothèse selon la revendication 3, **caractérisée en ce que** ledit relief est un nez (25), alors que lesdits moyens complémentaires comprennent une fourche (35) dont deux branches (35a, 35b) définissent entre elles en volume (35c) de réception et de coulissement (F₁) dudit nez.

5. Prothèse selon l'une des revendications précédentes, **caractérisée en ce que** lesdits moyens de guidage (25) et lesdits moyens de blocage (26) sont ménagés au voisinage de deux bords opposés (20a, 20b) de ladite seconde surface d'articulation (S₂), alors que lesdits moyens complémentaires (35) et les moyens de blocage (36) prévus sur ledit élément de verrouillage (30) sont ménagés sur deux bords opposés (30a, 30b) dudit élément de verrouillage (30).

6. Prothèse selon l'une des revendications précédentes, **caractérisée en ce que** lesdits moyens de blocage comprennent une vis (36) et un taraudage (26) complémentaires prévus respectivement sur ledit élément de verrouillage (30) et sur ledit composant cubital (20), ou réciproquement, et **en ce qu'**il est prévu des moyens (36b, 38) de blocage en rotation de ladite vis.

7. Prothèse selon la revendication 6, **caractérisée en ce que** la pièce (30) contre laquelle porte la tête (36a) de ladite vis (36) est pourvue d'une patte (38) repliable (F₃) en configuration de blocage de ladite tête.

8. Prothèse selon la revendication 7, **caractérisée en ce que** la tête (36a) de ladite vis (36) est à profil polygonal, avec des faces dont la largeur est supérieure à la largeur de ladite patte repliable (38).

9. Prothèse selon l'une des revendications 6 à 8, **caractérisée en ce que** la tête (36a) de ladite vis (36) est à section non circulaire, alors que la pièce (30) contre laquelle porte ladite tête forme un espace (37b) de réception de ladite tête, avec contact entre la surface (37c) de ladite pièce et la surface externe de ladite tête.

10. Prothèse selon la revendication 9, **caractérisée en ce que** ladite tête est pourvue de crans (36b) aptes à pénétrer superficiellement dans la surface (37c) de ladite pièce (30).

## Patentansprüche

1. Ellbogenprothese, die einen Humeralisbauteil umfasst, der eine erste Anlenkfläche bildet, und einen Cubitalisbauteil, der eine zweite Anlenkfläche bildet, die um einen Teil der ersten Anlenkfläche angeordnet werden und um eine Längsachse der ersten Anlenkfläche schwenken kann, wobei der Cubitalisbauteil mit Montagemitteln für ein Verriegelungselement versehen ist, das eine dritte Anlenkfläche bildet, die die zweite Anlenkfläche verlängert und ebenfalls um einen Teil der ersten Anlenkfläche angeordnet werden kann, **dadurch gekennzeichnet, dass** die Montagemittel (25, 26) Mittel (25) zum Führen umfassen, die mit komplementären Mitteln (35) zusammenarbeiten können, die auf dem Verriegelungselement (30) vorgesehen sind, um das Verriegelungselement bei Verschiebung (F₁) in Bezug auf den Cubitalisbauteil (20) bis zu einer Position (Figur 3) zu führen, in der Blockierungsmittel (36, 26), die jeweils auf dem Verriegelungselement und auf dem Cubitalisbauteil vorgesehen sind, betätigt (F₂) werden können, um das Verriegelungselement auf dem Cubitalisbauteil festzustellen, wobei die Blockierungsmittel nur in dieser Stellung betätigt werden können.

2. Prothese nach Anspruch 1, **dadurch gekennzeichnet, dass** die Blockierungsmittel eine Schraube (36) und eine Innengewindebohrung (26), die komplementär sind, umfassen, die jeweils auf dem Verriegelungselement (30) und auf dem Cubitalisbauteil (20) oder umgekehrt vorgesehen sind, wobei die Richtung der relativen Verschiebung (F₁) zwischen dem Element und dem Bauteil, wie sie von den Führungsmitteln (25, 23) auferlegt wird, zu der Längsachse (Y₁) der Innengewindebohrung und/oder der Schraube nicht parallel ist.

3. Prothese nach einem der vorhergehenden Ansprüche, **dadurch gekennzeichnet, dass** die Führungsmittel mindestens ein Relief (25) umfassen, das in Bezug auf einen Hauptteil (22a) des Cubitalisbauteils (20) vorragt, während die komplementären Mittel (35), die auf dem Verriegelungselement (30) vorgesehen sind, mit dem Relief durch Zusammenarbeiten von Formen in Wechselwirkung treten können.

4. Prothese nach Anspruch 3, **dadurch gekennzeichnet, dass** das Relief eine Nase (25) ist, während die komplementären Mittel eine Gabel (35) umfassen, deren zwei Zinken (35a, 35b) untereinander ein Volumen (35c) zur Aufnahme und zum Gleiten (F₁) der Nase definieren.

5. Prothese nach einem der vorhergehenden Ansprüche, **dadurch gekennzeichnet, dass** die Führungsmittel (25) und die Blockierungsmittel (26) in der Nähe von zwei entgegengesetzten Rändern (20a, 20b) der zweiten Anlenkfläche (S₂) eingerichtet sind, während die komplementären Mittel (35) und die Blockierungsmittel (36), die auf dem Verriegelungselement (30) vorgesehen sind, auf zwei entgegengesetzten Rändern (30a, 30b) des Verriegelungselements (30) angeordnet sind.

6. Prothese nach einem der vorhergehenden Ansprüche, **dadurch gekennzeichnet, dass** die Blockierungsmittel eine Schraube (36) und eine Innengewindebohrung (26) umfassen, die komplementär sind, die jeweils auf dem Verriegelungselement (30) und auf dem Cubitalisbauteil (20) vorgesehen sind oder umgekehrt, und dass Mittel (36b, 38) zum Blockieren der Schraube gegen Drehung vorgesehen sind.

7. Prothese nach Anspruch 6, **dadurch gekennzeichnet, dass** das Teil (30), gegen welches der Kopf (36a) der Schraube (36) aufliegt, mit einem Lappen (38) versehen ist, die in Blockierungskonfiguration des Kopfs rückklappbar (F₃) ist.

8. Prothese nach Anspruch 7, **dadurch gekennzeichnet, dass** der Kopf (36a) der Schraube (36) ein Vieleckprofil mit Seiten hat, deren Breite größer ist als die Breite des rückklappbaren Lappens (38).

9. Prothese nach einem der Ansprüche 6 bis 8, **dadurch gekennzeichnet, dass** der Kopf (36a) der Schraube (36) einen nicht kreisförmigen Querschnitt hat, während das Teil (30), gegen welches der Kopf aufliegt, einen Raum (37b) zur Aufnahme des Kopfes mit Berührung zwischen der Fläche (37c) des Teils und der äußeren Fläche des Kopfes bildet.

10. Prothese nach Anspruch 9, **dadurch gekennzeichnet, dass** der Kopf mit Rasten (36b) versehen ist, die oberflächlich in die Fläche (37c) des Teils (30) eindringen können.

## Claims

1. Elbow prosthesis comprising a humeral component forming a first articulation surface and an ulnar component forming a second articulation surface capable of being arranged around a part of the said first articulation surface and of pivoting about a longitudinal axis of the said first articulation surface, the said ulnar component being provided with means for mounting a locking element which forms a third articulation surface extending the second articulation surface and capable of likewise being arranged around a part of the said first articulation surface, **characterised in that** the said mounting means (25, 26) comprise guiding means (25) adapted to cooperate with complementary means (35) provided on the said locking element (30) to guide the said locking element in translation (F₁) with respect to the said ulnar component (20) as far as a position (Figure 3) in which locking means (36, 26) provided respectively on the said locking element and on the said ulnar component can be implemented (F₂) to immobilise the said locking element on the said ulnar component, the said locking means being able to be implemented only in the said position.

2. Prosthesis according to Claim 1, **characterised in that** the said locking means comprise a screw (36) and a tapping (26) which are complementary and provided respectively on the said locking element (30) and on the said ulnar component (20), or vice versa, the direction of relative translation (F₁) between the said element and the said component as imposed by the said guiding means (25, 23) being nonparallel to the longitudinal axis (Y₁) of the said tapping and/or the said screw.

3. Prosthesis according to one of the preceding claims, **characterised in that** the said guiding means comprise at least one protrusion (25) projecting with respect to a main part (22a) of the said ulnar component (20), while the complementary means (35) provided on the said locking element (30) are capable of interacting with the said protrusion by cooperation of shapes.

4. Prosthesis according to Claim 3, **characterised in that** the said protrusion is a nose (25), while the said complementary means comprise a fork (35), the two legs (35a, 35b) of which define between them a volume (35c) for reception and sliding (F₁) of the said nose.

5. Prosthesis according to one of the preceding claims, **characterised in that** the said guiding means (25) and the said locking means (26) are arranged in the vicinity of two opposite edges (20a, 20b) of the said second articulation surface (S₂), while the said complementary means (35) and the locking means (36) provided on the said locking element (30) are arranged on two opposite edges (30a, 30b) of the said locking element (30).

6. Prosthesis according to one of the preceding claims, **characterised in that** the said locking means comprise a screw (36) and a tapping (26) which are complementary and provided respectively on the said locking element (30) and on the said ulnar component (20), or vice versa, and **in that** there are provided means (36b, 38) for rotationally locking the said screw.

7. Prosthesis according to Claim 6, **characterised in that** the piece (30) against which the head (36a) of the said screw (36) bears is provided with a tab (38) which can be folded over (F₃) into a configuration for locking the said head.

8. Prosthesis according to Claim 7, **characterised in that** the head (36a) of the said screw (36) has a polygonal profile, with faces whose width is greater than the width of the said tab (38) which can be folded over.

9. Prosthesis according to one of Claims 6 to 8, **characterised in that** the head (36a) of the said screw (36) is of noncircular section, while the piece (30) against which the said head bears forms a space (37b) for receiving the said head, with contact between the surface (37c) of the said piece and the outer surface of the said head.

10. Prosthesis according to Claim 9, **characterised in that** the said head is provided with notches (36b) capable of penetrating superficially into the surface (37c) of the said piece (30).
